# EUROPEAN PATENT SPECIFICATION

(11) **EP 4 548 856 B1**
(45) Date of publication and mention of the grant of the patent: **28.10.2026**
(21) Application number: 25158273.0
(22) Date of filing: 10.06.2020
(51) Int. Cl.: A61B 17/00, A61B 17/04, A61B 17/062

(54) **APPARATUS FOR INCREASING FORCE DISTRIBUTION AT SUTURE-TISSUE INTERFACE**
VORRICHTUNG ZUR ERHÖHUNG DER KRAFTVERTEILUNG AN DER NAHT-GEWEBE-SCHNITTSTELLE
APPAREIL POUR AUGMENTER LA DISTRIBUTION DE FORCE AU NIVEAU D'UNE INTERFACE FIL DE SUTURE-TISSU

(30) Priority: 11.06.2019 US 201962860108 P
(43) Date of publication of application: 07.05.2025
(62) Document number(s) of the earlier application(s) in accordance with Art. 76 EPC: 20751355.7 / 3 934 543
(73) Proprietor: Boston Scientific Scimed, Inc., Maple Grove, MN 55133 (US)
(72) Inventor: CHRISTAKIS, Laura E., Framingham, Massachusetts 01702 (US); SMITH, Paul, Smithfield, Rhode Island 02917 (US); WALES, Ryan V., Northborough, Massachusetts 01532 (US); BRECHBIEL, Scott E., Acton, Massachusetts 01720 (US); GRAY, Jeff, Sudbury, Massachusetts 01776 (US); FLEURY, Sean P., Minneapolis, Minnesota 55404 (US)
(74) Representative: Vossius & Partner Patentanwälte Rechtsanwälte mbB

(56) References cited:
- WO-A1-2019/108261
- WO-A2-2005/086945
- US-A1- 2002 193 816
- US-A1- 2005 251 153
- US-A1- 2007 049 929
- US-A1- 2011 118 758

## Description

### CROSS-REFERENCE TO RELATED APPLICATIONS

The present application claims the benefit of priority under 35 U.S.C. §119 to U.S. Provisional Patent Application 62/860,108, filed June 11, 2019.

### FIELD

The present disclosure relates generally to suturing devices and, more particularly, to suturing devices for increasing force distribution at a suture-tissue interface.

### BACKGROUND

Suturing is used during a wide variety of endoscopic procedures, such as closing a wound, anchoring a stent or other implant, or shorting curvature in the stomach. Several types of sutures and devices for implantation and extraction have been developed. In some approaches, suturing is performed by repeatedly passing a sharp suture needle attached to a length of suture material through portions of tissue to be sutured together. The free ends of the suture material are then tied together to complete the suturing procedure.

Many endoscopic procedures pull tissue together and hold the tissue in a new configuration. However, reconfiguring and constraining tissue generates tension between the tissue and the suture. While current sutures and suturing approaches may provide an adequate temporary solution to holding tissue, over time the force required to hold the tissue in place becomes too great to sustain an equilibrium. As a result, when the tissue relaxes the suture can tear through the tissue. It is with the above considerations in mind that the improvements of the present disclosure may be useful.
WO 2005/086945 A2 discloses endoluminal tool deployment systems.

### SUMMARY

The present disclosure in its various embodiments relates generally to apparatuses, devices, and methods for redistributing force at the tissue-suture interface to increase long-term effectiveness of an endoscopic suturing procedure. The invention is defined in appended claim 1.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. Furthermore, some of the figures include cross-sectional views in the form of "slices", or "near-sighted" cross-sectional views, omitting certain background lines or features otherwise visible in a "true" cross-sectional view, for illustrative clarity. In the figures:
**FIG. 1** is a side view of a tissue suturing apparatus according to embodiments of the present disclosure;
**FIG. 2** is a side cross-sectional view of an example deployment device of the tissue suturing apparatus of **FIG. 1****,** according to embodiments of the present disclosure;
**FIG. 3A** is a front view of an example covering over the deployment device of **FIG. 2****,** according to embodiments of the present disclosure;
**FIG. 3B** is a front view of an example covering over the deployment device of **FIG. 2****,** according to embodiments of the present disclosure;
**FIG. 4A** is an end view of an example deployment device of the tissue suturing apparatus of **FIG. 1****,** according to embodiments of the present disclosure;
**FIGS. 4B-4E** are side cross-sectional views demonstrating a process for using the deployment device of **FIG. 4A****,** according to embodiments of the present disclosure;
**FIG. 5** is a perspective view of an example deployment device according to embodiments of the present disclosure;
**FIG. 6** is a partial cross-sectional perspective view of the deployment device of **FIG. 5****,** according to embodiments of the present disclosure;
**FIGS. 7A-7I** are side views demonstrating a process for using the deployment device of **FIGS. 5-6****,** according to embodiments of the present disclosure;
**FIGS. 8A-8D** are perspective views demonstrating use of an example suture according to embodiments of the present disclosure;
**FIGS. 9A-9D** are perspective views demonstrating use of an example suture according to embodiments of the present disclosure;
**FIG. 10A** is a side view of an example suture according to embodiments of the present disclosure;
**FIG. 10B** is a top cross-sectional view of the suture of **FIG. 10A****,** according to embodiments of the present disclosure;
**FIG. 10C** is a side view of the suture of **FIG. 10A** following retraction of the suture, according to embodiments of the present disclosure;
**FIG. 11A** is a top cross-sectional view of an example suture according to embodiments of the present disclosure;
**FIG. 11B** is a side view of the suture of **FIG. 11A** following retraction of the suture, according to embodiments of the present disclosure;
**FIGS. 12A-12B** are perspective views demonstrating use of a sprayable adhesive applied to an area of tissue according to embodiments of the present disclosure;
**FIGS. 13A-13C** are perspective views demonstrating use of a glue applied to an area of tissue according to embodiments of the present disclosure;
**FIG. 14A** is a perspective view demonstrating use of tubular tissue supports according to embodiments of the present disclosure;
**FIG. 14B** is a perspective view an example tubular tissue support according to embodiments of the present disclosure;
**FIG. 14C** is a perspective view another example tubular tissue support according to embodiments of the present disclosure;
**FIG. 15** is a perspective view illustrating the use of spring-loaded wires according to embodiments of the present disclosure; and
**FIG. 16** is a flow diagram of a method according to embodiments of the present disclosure.

### DETAILED DESCRIPTION

Embodiments herein disclose endoscopic tissue suturing apparatuses, devices, and methods for increasing force distribution at a suture-tissue interface. Those skilled in the art will appreciate that while the approaches of the present disclosure will herein be described with reference to suturing an area of the stomach, the approaches may be utilized in other gastrointestinal transluminal procedures, and may be introduced transorally as well as transanally.

Although non-limiting, approaches of the present disclosure may be effective for treating gastroesophageal reflux disease (GERD) by performing procedures such as partial circumferential endoscopic mucosal resection (EMR) or submucosal dissection (ESD) of a patient's gastrointestinal tract.

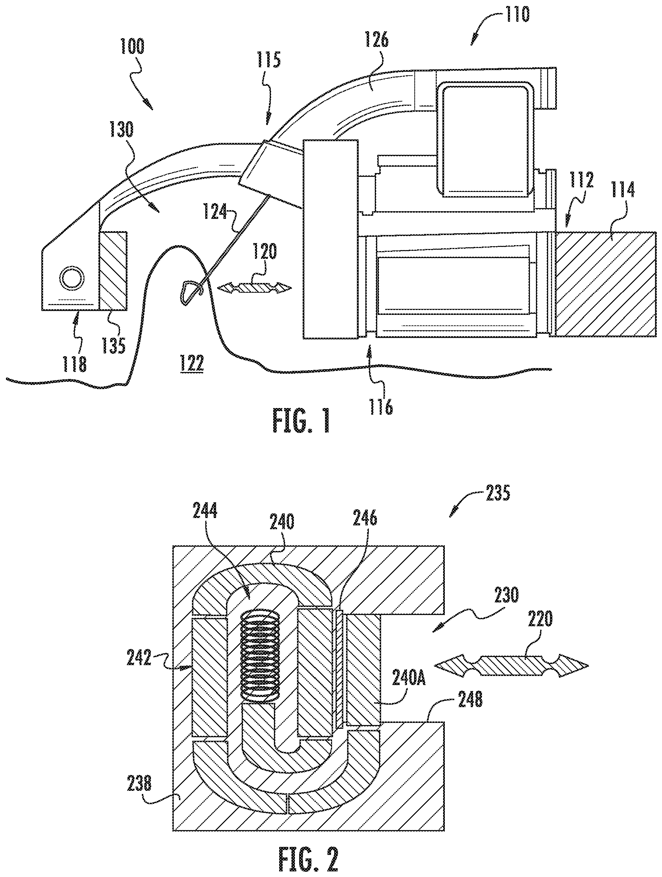

Turning now to **FIG. 1****,** a tissue suturing apparatus (hereinafter "apparatus") 100 according to embodiments of the present disclosure will be described. As shown, the apparatus 100 includes a tissue suturing device (hereinafter "device") 110 extending from a distal end 112 of a scope, such as an endoscope 114. The device 110 may be used with other types of scopes as well, such as bronchoscopes, duodenoscopes, gastroscopes, colonoscopes, etc. The device 110 may include a head or cap 115 having a fixed end 116 directly coupled to the endoscope 114, and a free end 118 opposite the fixed end 116. In some embodiments, the cap 115 may be coupled to the endoscope 114 using a variety of techniques, such as by mechanical fasteners, glue, suture, press fit, tape, overmolding, etc. In other embodiments, the dispenser cap may be integrally formed with the endoscope.

During use, a needle 120 may be passed back and forth between the fixed end 116 and the free end 118 along a longitudinal axis, the needle 120 including one or more pointed tips for puncturing a target tissue 122 with each pass. In some embodiments, a tissue grasper 124 may be delivered through a channel 126 of the cap 115 to engage the target tissue 122, bringing the target tissue 122 into a suture cavity 130 located between the fixed end 116 and the free end 118.

The device 110 includes a deployment device 135 containing a plurality of tissue supports (not shown) therein. As will be explained in greater detail below, the tissue supports may be added between a suture and the target tissue 122 during one or more passes of the needle 120. In some embodiments, the deployment device 135 may be coupled to the free end 118 of the device 110, positioned generally opposite the fixed end 116.

Turning now to **FIG. 2****,** an example deployment device 235 according to embodiments of the present disclosure will be described. As shown, the deployment device 235 is a cartridge including a housing 238 containing a plurality of tissue supports 240 therein. The housing 238 may include an interior channel 242 and a biasing device 244 (e.g., a spring) for biasing the plurality of tissue supports 240 through the interior channel 242. In some embodiments, the plurality of tissue supports 240 are solid, flexible components arranged end-to-end. During suturing, a needle 220 enters a suture cavity 230, puncturing an exposed tissue support 240A of the plurality of tissue supports 240. As the exposed tissue support 240A is brought away from the deployment device 235, attached to the needle 220, the biasing device 244 may cause the next tissue support of the plurality of tissue supports 240 to enter the suture cavity 230 for subsequent engagement by the needle 220 on the next pass. One or more of the plurality of tissue supports 240 may be added every time the needle 220 passes through the tissue such that the interface between a suture and the tissue becomes more robust as the procedure continues. In some embodiments, the deployment device 235 may include a rigid plate 246 positioned behind the exposed tissue support 240A to prevent the needle 220 from extending into the housing 238. Furthermore, the deployment device 235 may include an overhang 248, which may keep the exposed tissue support 240A aligned within the suture cavity 230.

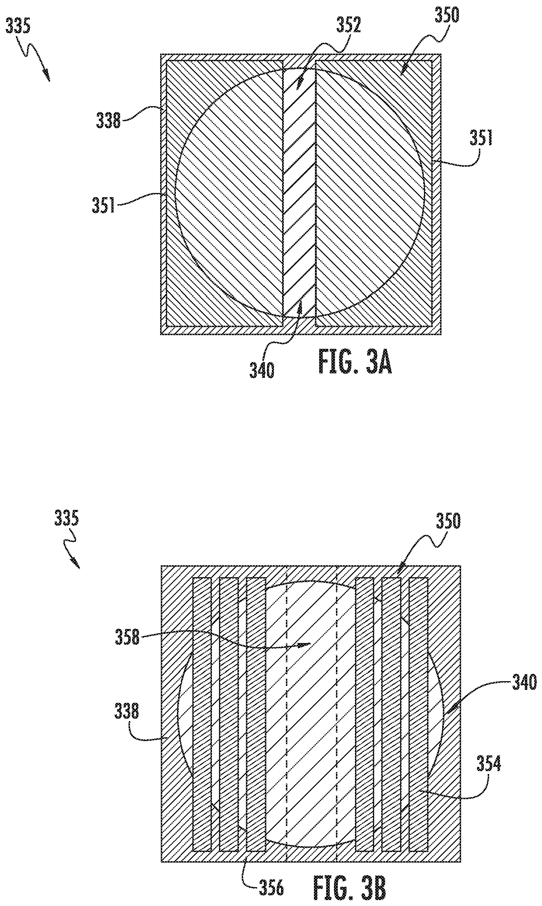

Turning now to **FIGS. 3A-3B****,** various deployment devices according to embodiments of the present disclosure will be described in greater detail. As shown, a deployment device 335 may include a plurality of tissue supports 340 stacked atop one another within a housing 338. The deployment device 335 includes a cover 350 extending across the housing 338. In the embodiment of **FIG. 3A****,** the cover 350 may include one or more membranes 351 extending across the housing 338. The membrane(s) 351 define an opening 352 to permit access to the plurality of tissue supports 340 by a needle (not shown). The membrane(s) 351 may be durable enough to hold the plurality of tissue supports 340 in place within the deployment device 335, yet flexible enough to allow an exposed tissue support of the plurality of tissue supports 340 to be pulled from the deployment device 335 by the needle.

In the embodiment of **FIG. 3B****,** the cover 350 may include a plurality of components, such as slats 354 extending across a frame 356 of the housing 338 to hold the plurality of tissue supports 340 in place. In various examples, the plurality of slats 354 may be oriented laterally, longitudinally and/or diagonally. As shown, the plurality of slats 354 may define an opening 358 to allow a needle to enter the deployment device 335 and puncture an exposed tissue support of the plurality of tissue supports 340. The exposed tissue support may then be folded or bent, and then pulled through the opening 358.

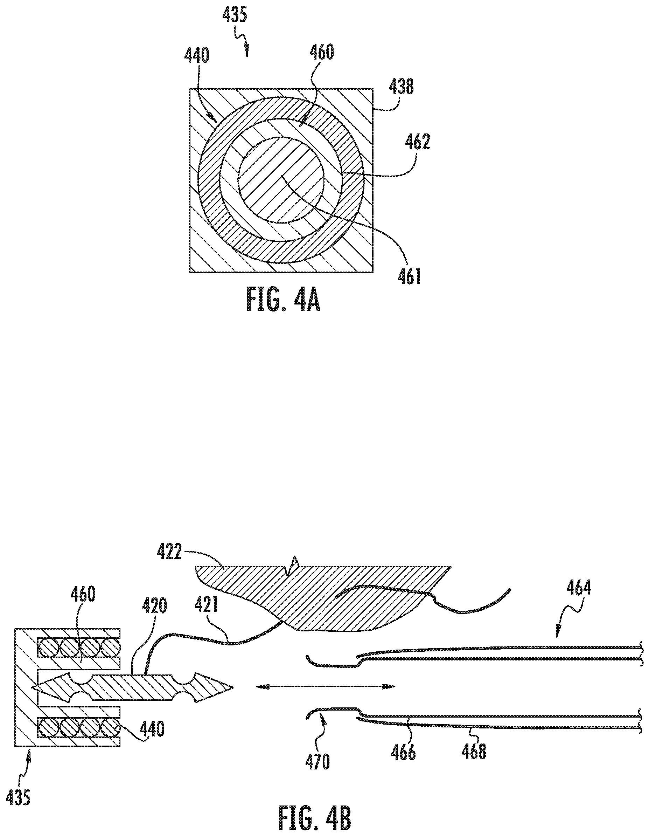

As shown in **FIG. 4A****,** a deployment device 435 may include a mandrel 460 within a housing 438. In some embodiments, the mandrel 460 may be a tubular shaped component defining a central channel 461 therein. As will be described in greater detail below, the central channel 461 permits a needle to enter the deployment device 435 and retain one or more of a plurality of tissue supports 440. In this embodiment, the plurality of tissue supports 440 may be cylindrical washers disposed concentrically around an exterior surface 462 of the mandrel 460. Furthermore, the plurality of tissue supports 440 may elastically expand to fit the mandrel 460 in the deployment device 435. After deployment, the plurality of tissue supports 440 may shrink or constrict to effectively close around a suture.

Turning now to **FIGS. 4B-4E****,** cross-sectional views demonstrating operation of the deployment device 435 of **FIG. 4A** will be described in greater detail. As shown in **FIG. 4B****,** the deployment device 435 contains a plurality of tissue supports 440 (e.g., four) disposed around the mandrel 460. The deployment device 435 may operate with a needle shuttling system 464, which may include two or more nested hypo-tubes, such as an inner tube 466 and an outer tube 468 operable to shuttle a needle 420 and a suture 421 back and forth through a target tissue 422. In some embodiments, a set of prongs 470 may extend from the inner tube 466. In various examples, the inner tube 466 and the outer tube 468 may move together or independently.

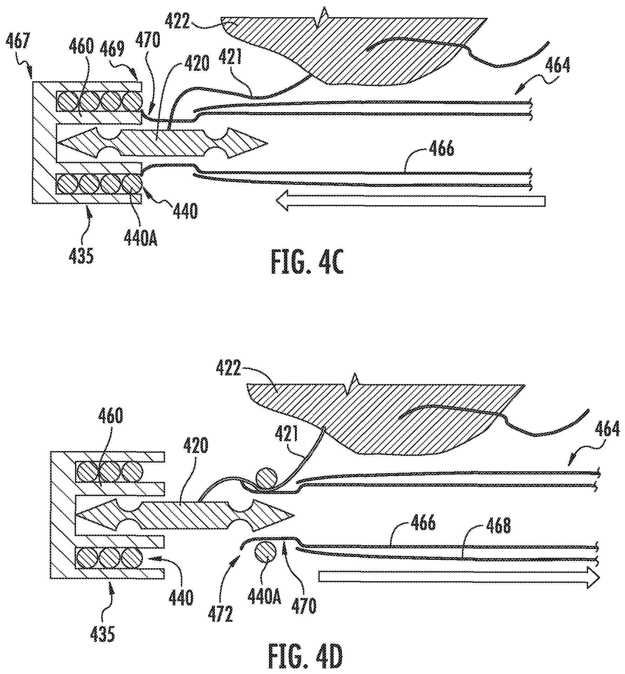

As shown in **FIG. 4C****,** the inner tube 466 of the needle shuttling system 464 may be passed distally over the needle 420 to interact with the deployment device 435. For example, the prongs 470 on the end of the inner tube 466 may be deformable to fit alongside/adjacent the plurality of tissue supports 440. In some embodiments, a depth that the prongs 470 enter the deployment device 435 can be controlled at the handle (not shown) by an operator. In other embodiments, the prongs 470 merely abut a proximal end 469 of the deployment device 435. Although not shown, the deployment device 435 may include a spring system (e.g., one or more springs, or other biasing mechanisms) incorporated into a distal end 467 of the deployment device 435 to continually or selectively push the plurality of tissue supports 440 to the proximal end 469 for engagement with the prongs 470. For example, in response to the needle being received in the deployment device 435, a proximal-most tissue support 440 may be deployed onto the prongs 470.

When the needle shuttling system 464 is retracted from the deployment device 435, an outermost tissue support 440A of the plurality of tissue supports 440 may be removed from the mandrel 460, for example, as shown in **FIG. 4D****.** In some embodiments, the outermost tissue support 440A may extend around the suture 421 and remain on the prongs 470 while the inner tube 466 moves farther away from the deployment device 435. The outermost tissue support 440A may contract, thereby remaining on the prongs 470 by tension. Furthermore, the prongs may include one or more curved ends 472 (e.g., curved radially outward) to prevent the outermost tissue support 440A from sliding off the prong 470.

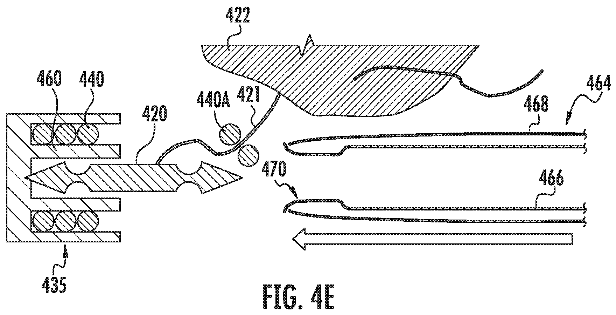

Next, as shown in **FIG. 4E****,** the outer tube 468 of the needle shuttling system 464 may be advanced distally in relation to the inner tube 466, pushing the outermost tissue support 440A off the prongs 470 and onto the suture 421, for example, between the needle 420 and the target tissue 422. The position of the outer tube 468 can then be reset in relation to the inner tube 466 so the needle shuttling system 464 is ready for another needle pass. In some embodiments, a tissue support may not be added in between each suture pass. Furthermore, it will be appreciated that the number of tissue supports deployed depends on the needs of the procedure, physician, and/or the capacity of the deployment device 435. Embodiments herein are not limited in this context.

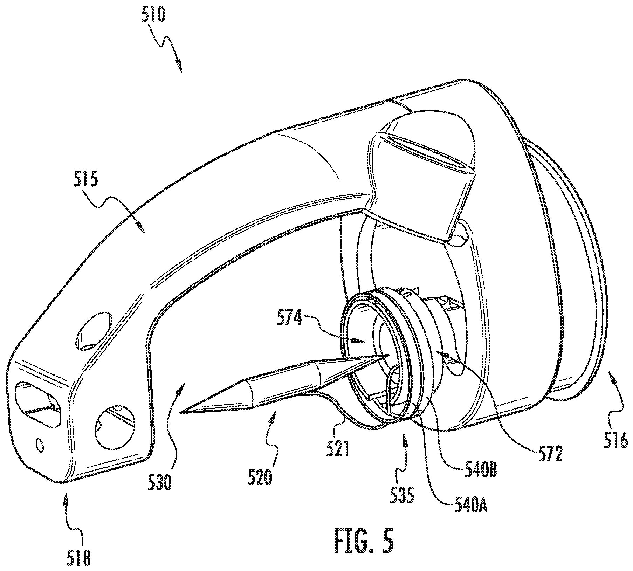

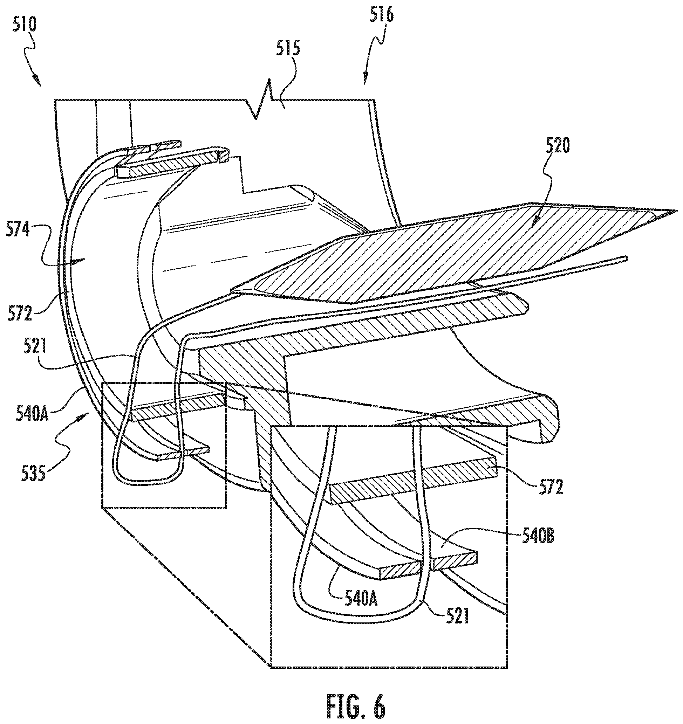

Turning now to **FIGS. 5-6****,** a tissue suturing device (hereinafter "device") 510 according to embodiments of the present disclosure will be described. The device 510 may include a head or cap 515 having a fixed end 516 coupleable with an endoscope (not shown), and a free end 518 opposite the fixed end 516. In some embodiments, the cap 515 may be integrally formed with an endoscope or other instrument. During use, a needle 520 and a suture 521 may be passed back and forth between the fixed end 516 and the free end 518 along a longitudinal axis, puncturing a target tissue (not shown) positioned in a suture cavity 530.

As further shown, the device 510 may include a deployment device 535 coupled to the fixed end 516 of the cap 515. In this embodiment, the deployment device 535 may include a plurality of tissue supports 540A-540B extending around a collar 572. During assembly, the suture 521, which is connected to the needle 520, may be threaded between tissue support 540A and tissue support 540B to allow distal most positioned tissue support 540A to be placed between the needle 520 and the tissue.

As shown, the collar 572 may secure the device 510 onto a distal end of the endoscope. In other embodiments, the collar 572 is integrally formed with the endoscope or other tubular instrument. Although non-limiting, each of the plurality of tissue supports 540A-540B may be a washer-shaped support disposed around an exterior of the collar 572. The collar 572 may define a collar opening 574 aligned with a working channel of the endoscope. The needle 520 may pass in and out of the collar opening 574 during suturing, as will be described in greater detail below. While situated on the collar 572, the plurality of tissue supports 540 are stretched for deployment over the needle 520 and the suture 521. As the suture 521 is pulled at the proximal handle of the device 510, each of the plurality of tissue supports 540 may be deployed separately. Advantageously, the plurality of tissue supports 540A-540B may be deployed on opposite sides of the target tissue being tensioned together, for example, after the operator has passed the needle 520 through the target tissue a desired number of times but before the suture 521 is cinched.

In some embodiments, the plurality of tissue supports 540 are deployed using an additional suture (not shown). For example, the additional suture may be connected to one or more of the plurality of tissue supports 540A-540B. As the additional suture is pulled towards the fixed end 516, the additional suture may release distal most positioned tissue support 540A from the collar 572. In some embodiments, the collar 572 may include an aperture (not shown) through a sidewall thereof, the aperture allowing the additional suture to exit from the central bore of the collar 572. Having the plurality of tissue supports 540A-540B situated on the collar 572 with the additional suture may allow the needle 520 to shuttle back and forth normally as many times as the operator needs.

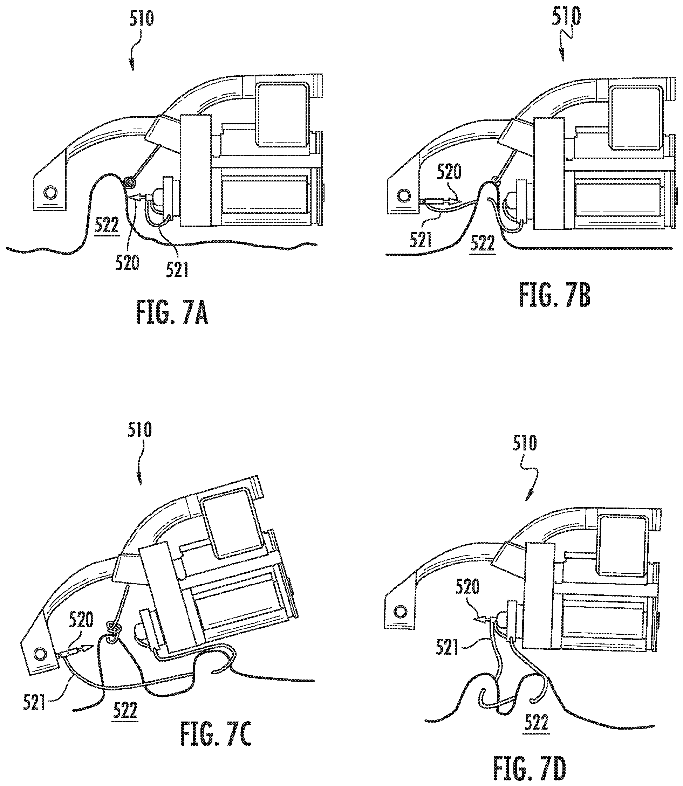

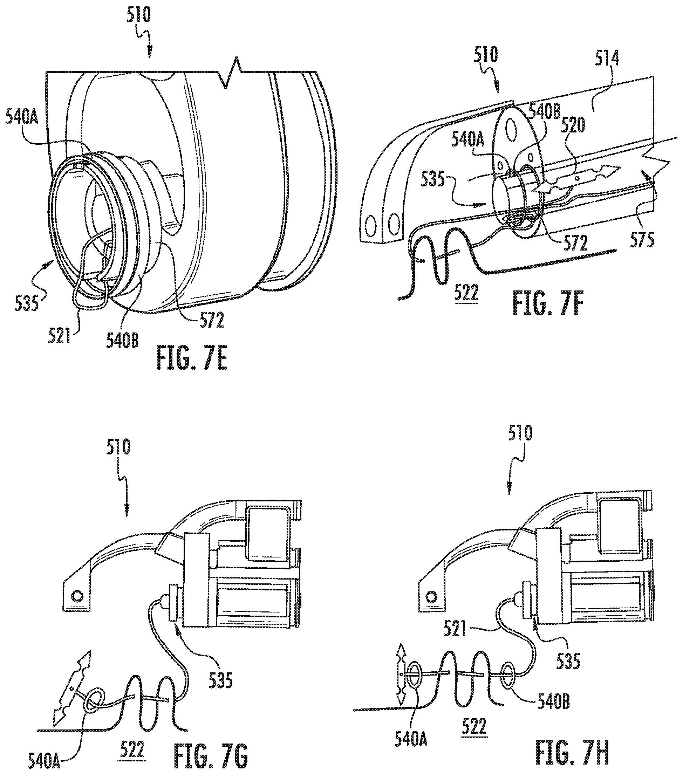

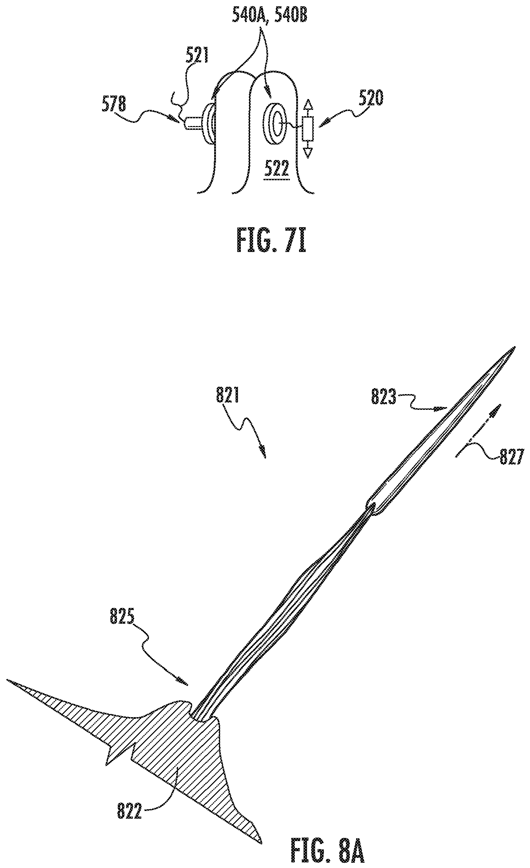

Turning now to **FIGS. 7A-7H****,** operation of the device 510 according to embodiments of the present disclosure will be described in greater detail. In **FIGS. 7A-7B****,** the needle 520 and the suture 521 may be passed through a first piece of the target tissue 522, and then through a second piece of the target tissue 522, as demonstrated in **FIGS. 7C-7D****.** As shown in **FIGS. 7E-****7F,** the needle 520 may then be pulled through the collar 572 of the deployment device 535 and into a working channel 575 of an endoscope 514. In some embodiments, the suture 521 may extend around just the distal most positioned tissue support 540A. During deployment, as demonstrated in **FIG. 7G****,** the distal most positioned tissue support 540A may be released from the deployment device 535 and then brought into position on one side of the target tissue 522. A second tissue support 540B may then be released from the deployment device 535, and then brought into position on an opposite side of the target tissue 522, as shown in **FIG. 7H****.** In some embodiments, the endoscope 514 **(****FIG. 7F****)** may be move away from the target tissue 522to bring the needle 520 into contact with one side of the target tissue 522, while the suture 521 pulled to deploy the second tissue support 540B onto the suture 521. Finally, as shown in **FIG. 7I****,** the suture 521 may be pulled tight and secured, for example, using a cinching device 578. The cinching device 578 may bring the distal most positioned tissue support 540A and second tissue support 540B on opposite sides of the target tissue 522 closer together by pulling the suture 521 to bring the needle 520 towards the deployment device 535.

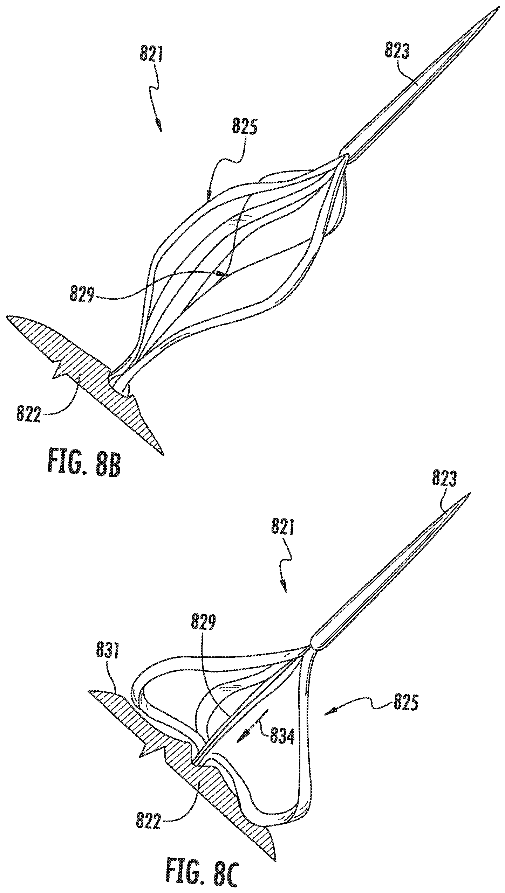

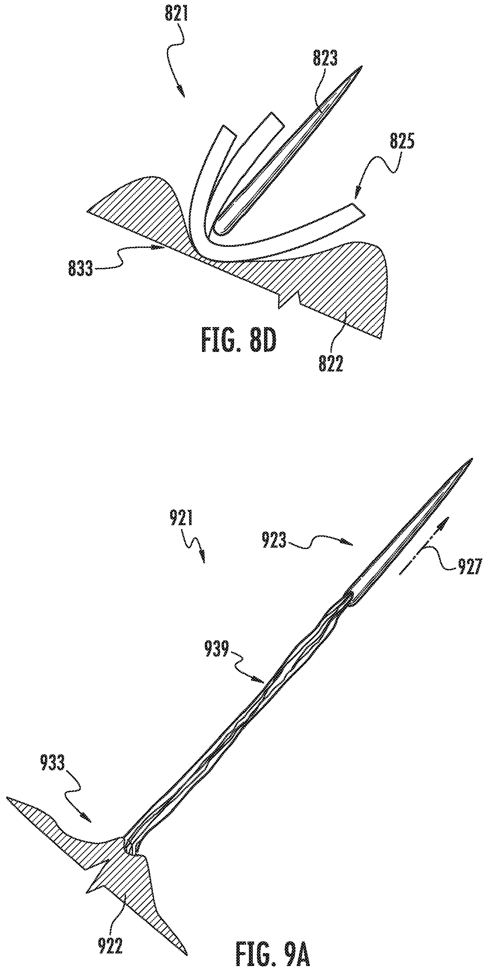

Turning now to **FIGS. 8A-8D****,** a suture 821 according to embodiments of the present disclosure will be described. The suture 821 may include a rigid or semi-rigid tip 823 operable to pierce a target tissue 822. The rigid tip 823 may be connected to a plurality of flexible strands or filaments 825. As shown in **FIG. 8A****,** the suture 821 may initially pass through the target tissue 822 in a direction demonstrated by arrow 827. In this configuration, the filaments are generally straight and elongated. As demonstrated in **FIG. 8B****,** the suture 821 may include one or more retention wires 829 surrounding the filaments 825. The retention wires 829 may be used to pull the suture 821 back towards the target tissue 822, for example, in a direction shown by arrow 834 in **FIG. 8C****.** Pulling the suture 821 toward the target tissue 822 causes the filaments 825 to extend radially, creating a malecot configuration against a surface 831 of the target tissue 822. As shown in **FIG. 8D****,** an interface 833 between the filaments 825 and the target tissue 822 may generally have larger area due the expanded configuration of filaments 825. Force is therefore better distributed at the interface 833, making it less likely that the target tissue 822 will rupture.

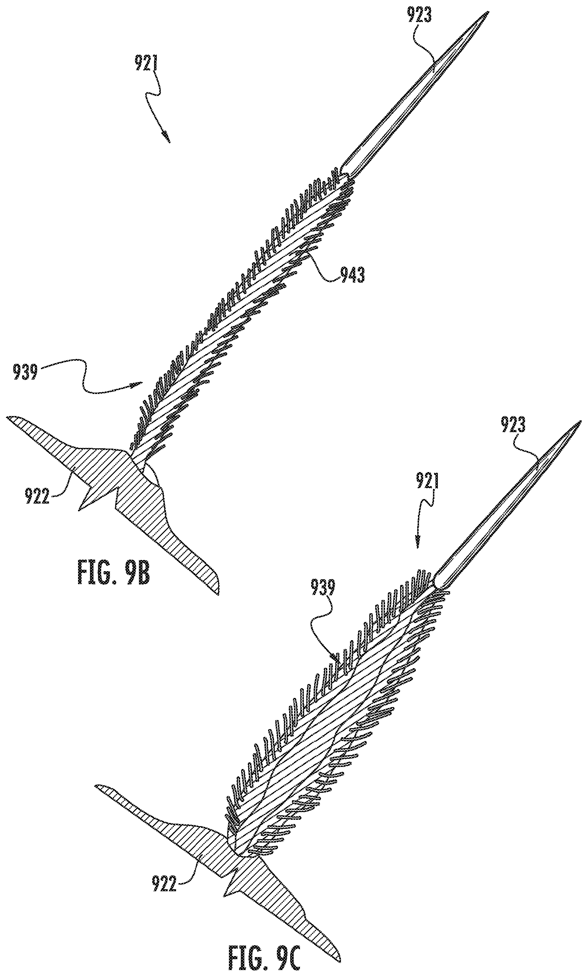

Turning now to **FIGS. 9A-9D****,** a suture 921 according to embodiments of the present disclosure will be described. The suture 921 may include a rigid tip 923 connected to a polymer body 939, wherein the polymer body 939 may be rigid or flexible. As shown in **FIG. 9A****,** the suture 921 may initially pass through at an interface 933 of the target tissue 922 in a direction shown by arrow 927. In this configuration, the polymer body 939 is generally straight and elongated. However, as demonstrated in **FIG. 9B****,** an outer material 943 of the polymer body 939 may expand or grow radially when it comes into contact with a fluid (not shown), such as water, saline, blood, etc.

In some embodiments the outer material 943 of the polymer body 939 may be a hydrogel. The term "hydrogel" may indicate a crosslinked, water insoluble, water containing material. Suitable cross-linkable polymers include but are not limited to one or a mixture of polymers selected from the group consisting of polyhydroxy ethyl methacrylate, polyvinyl alcohol, polyacrylamide, poly (N-vinyl pyrrolidone), polyethylene oxide, hydrolysed polyacrylonitrile, polyacrylic acid, polymethacrylic acid, polyethylene amine, alginic acid, pectinic acid, carboxy methyl cellulose, hyaluronic acid, heparin, heparin sulfate, chitosan, carboxymethyl chitosan, chitin, pullulan, gellan, xanthan, carboxymethyl starch, carboxymethyl dextran, chondroitin sulfate, cationic guar, cationic starch as well as salts and esters thereof. The hydrogel produces may have having improved mechanical properties, such as improved stiffness, modulus, yield stress, strength, etc., at the interface 933.

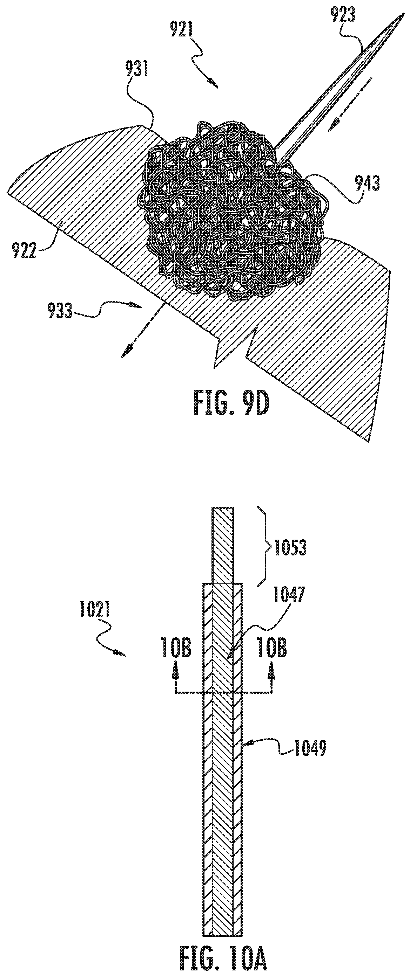

As shown in **FIG. 9C****,** as the suture 921 is retracted, the outer material 943 of the polymer body 939 begins to bunch. Further retraction of the suture 921 towards the target tissue 922, as demonstrated in **FIG. 9D****,** causes the outer material 943 to gather as a bundle along a surface 931 of the target tissue 922. The interface 933 between the outer material 943 and the target tissue 922 generally has a larger area due to the expansion of the outer material 943. Force is therefore better distributed at the interface 933, making it less likely that the target tissue 922 will rupture.

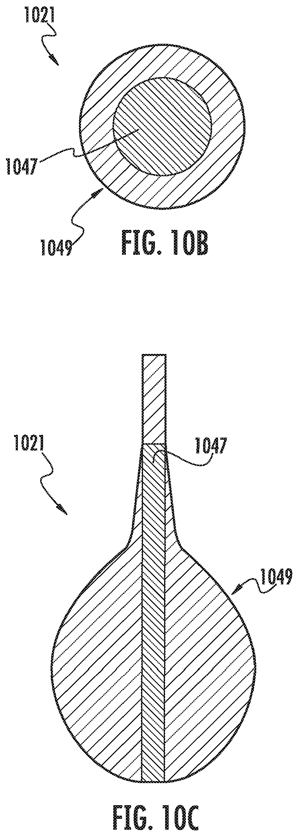

Turning now to **FIGS. 10A-10C****,** a suture 1021 according to embodiments of the present disclosure will be described. As shown, the suture 1021 may include a core 1047 surrounded by a sheath 1049. A portion of the core 1047 extending outside of the sheath 1049 may correspond to a tip 1053 of the suture 1021. In some embodiments, the sheath 1049 is capable of expanding radially from the core 1047. Furthermore, the sheath 1049 may move independent of the core 1047 to allow for bunching of the sheath, as demonstrated in **FIG. 10C****.** In this embodiment, the sheath 1049 may be a continuous or sheet-like covering that bulbs and buckles upon expansion and retraction of the core 1047, creating a solid plug-like interface where the suture 1021 meets a target tissue (not shown). In some embodiments, the sheath 1049 is disposed concentrically around the core 1047.

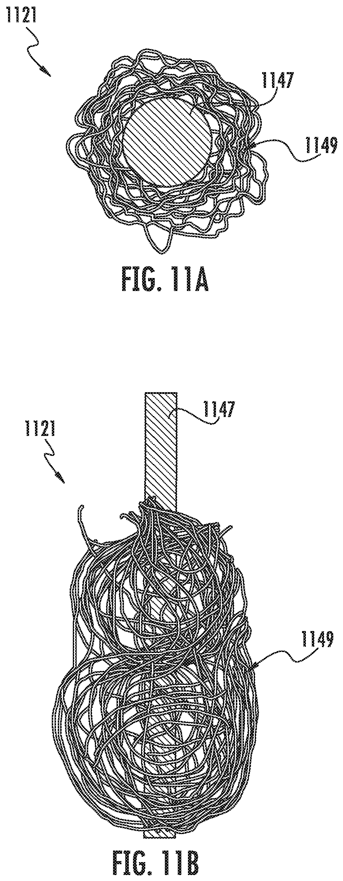

In the embodiment of **FIGS. 11A-11B****,** a sheath 1149 of a suture 1121 may include a plurality of flexible strands. As shown, as a core 1147 of the suture 1121 is retracted, the flexible strands bunch-up, expanding to create a mesh-like network with increased surface area. In some embodiments, the sheath 1149 is disposed concentrically around the core 1147.

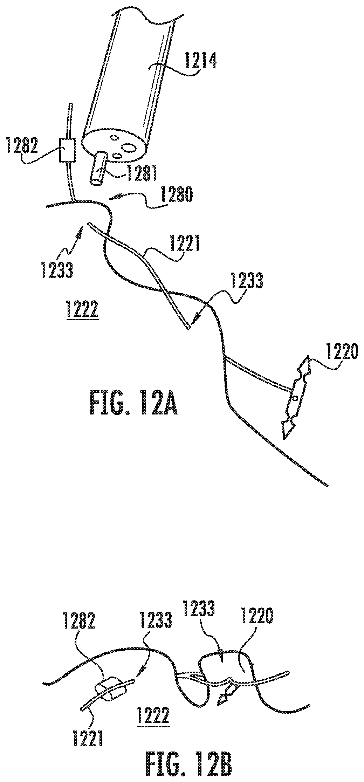

Turning now to **FIGS. 12A-12B****,** the use of a sprayable adhesive 1280 applied to an area of target tissue 1222 according to embodiments of the present disclosure will be described in greater detail. As shown, a sprayable adhesive 1280 may be delivered through an endoscope 1214. The sprayable adhesive 1280 may be applied to the target tissue 1222 at each interface 1233 where the suture 1221 and the needle 1220 penetrate the target tissue 1222. As a result, the interface 1233 may be less susceptible to tearing under the pull of the suture 1221.

During use, a physician may conduct an endoscopic procedure. The physician may retract a device occupying the working channel (e.g., visualization device) and advance a needle or catheter 1281. Once this feature reaches the working channel, a syringe (not shown) with a medical grade, silicone-based spray adhesive, for example, would be coupled to the proximal end of the catheter 1281 (e.g., via a Luer connector). The syringe may be actuated, forcing the sprayable adhesive 1280 to eject from the distal end of the endoscope 1214. As shown in **FIG. 12B****,** as the sprayable adhesive sets, the surface of the target tissue 1222 that a cinch 1282 and needle 1220 are in contact with will harden and prevent expansion, thus preventing the cinch 1282 and/or needle 1220 from passing through the hole in the target tissue 1222 at each interface 1233.

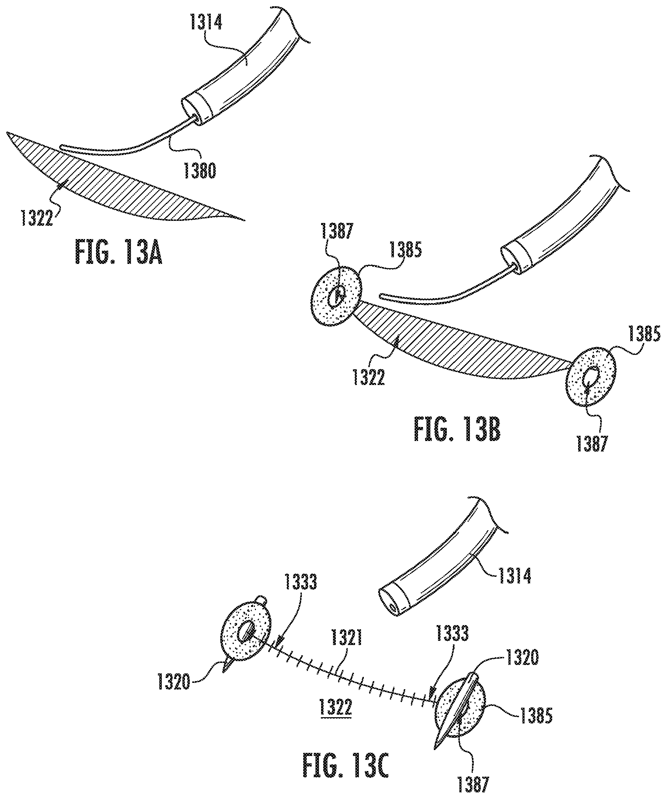

In the non-limiting embodiment of **FIGS. 13A-13C****,** a curable glue 1380 (e.g., moisture or UV curable) may be added to a target tissue 1322 at each end of a suture 1321 to distribute force applied on an interface 1333 between the target tissue 1322 and the suture 1321. Due to the flexible nature of the curable glue 1380 prior to curing, the curable glue 1380 can more easily pass through the working channel of the endoscope 1314 and be applied at the target tissue 1322. In addition, the curable glue 1380 may allow the physician to better customize a final shape for the curable glue 1380 based on the demands of the procedure, location of the wound, his/her preference, etc. For example, as shown in **FIGS. 13B-13C****,** the curable glue 1380 may be fashioned into one or more cylindrical washers 1385 each having a central opening 1387. As a result, the suture 1321 **(****FIG. 13C****)** may pass through the central openings 1387. Furthermore, use of the curable glue 1380 also gives the physician the freedom to add additional glue at various points in the procedure, for example, to improve the force distribution as needed pre- or post-suturing. In some embodiments, as shown in **FIG. 13C****,** the needle 1320 may remain on top of the curable glue 1380 following suturing. To improve visibility, the curable glue 1380 can also be dyed in alternative embodiments.

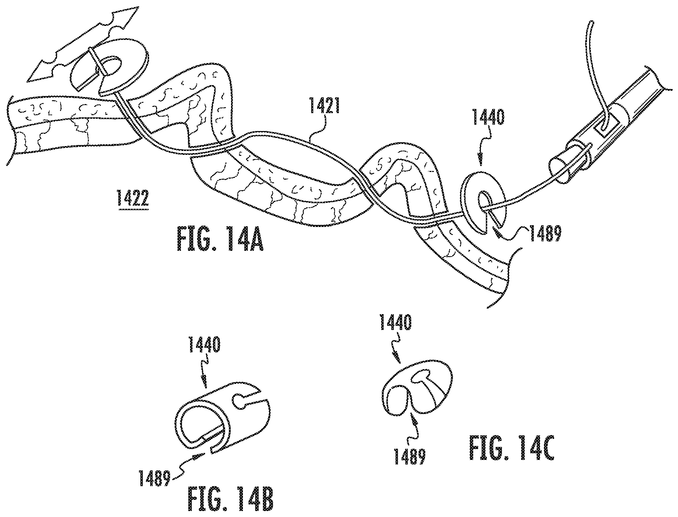

In the non-limiting embodiment of **FIGS. 14A-14C****,** one or more tubular tissue supports (e.g., washers) 1440 may be used on opposite sides of a target tissue 1422. As shown, the tubular tissue supports 1440 may include a slot 1489 through which a suture 1421 can run, allowing the tubular tissue supports 1440 to be placed anywhere along the suture 1421 after the suture 421 is threaded through the target tissue 1422. In some embodiments, the tubular tissue supports 1440 may be delivered endoscopically and maneuvered into place using a grasper or similar tool to protect the target tissue 1422 after the suture 1421 is placed, but before the suture 1421 is cinched.

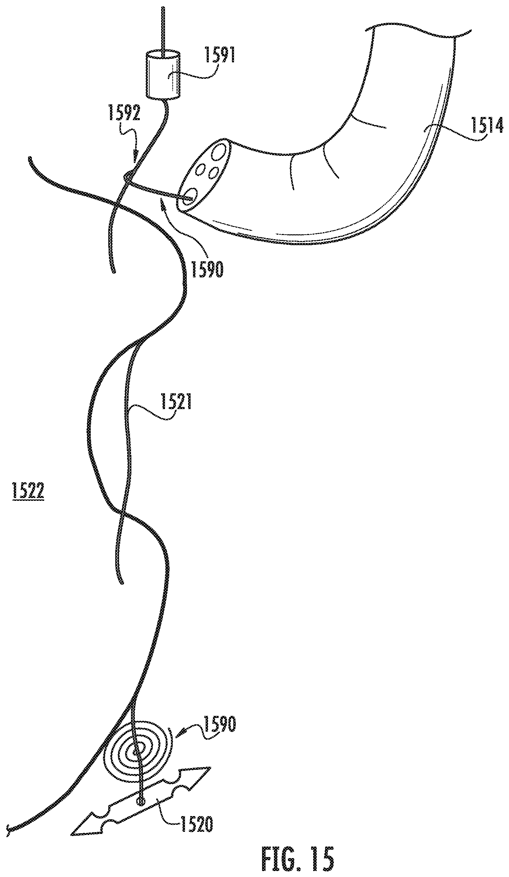

Turning now to **FIG. 15****,** tissue supports in the form of one or more elastic elements or spring-loaded wires will be described. As shown, a wire 1590 may be deployed from an endoscope 1514 and onto a suture 1521 as a needle 1520 and the suture 1521 traverse through a target tissue 1522. In the non-limiting embodiment shown, the wire 1590 may be a metallic, spring-loaded wire with shape memory, which is preloaded into a delivery catheter of the endoscope 1514. As the wire 1590 is advanced beyond a distal tip of the endoscope 1514, it begins to curl. The wire 1590 may create a hook 1592 as it begins to curl, the hook being engageable with the suture 1521 between the cinch 1591 and the target tissue 1522, and/or between the needle 1520 and the target tissue 1522.

Once the suture 1521 is acquired by the hook 1592, the rest of the wire 1590 may be fed out of the endoscope 1514. The wire 1590 continues to curl until a proximal end is expelled. Use of the wire 1590 allows for a low-profile during delivery and, once deployed, a large solid surface area for the cinch 1591 and the needle 1520 to interface with, thus preventing pull through. In non-limiting embodiments, the wire 1590 may be made of steel, Nitinol or any other material with a high spring constant. In other embodiments, the wire 1590 may also take on the profile of a ribbon, which may also increase a contact surface area between the wire 1590 and the cinch 1591 and the needle 1520.

Alternatively, the wire 1590 could be a bi-stable spring that is straight in one orientation and curved in another. The wire 1590 may be advanced until it is positioned between the cinch 1591 and the needle 1520. Once positioned appropriately, the spring may be shifted to a second orientation, causing it to curve into a coil or spiral-like orientation.

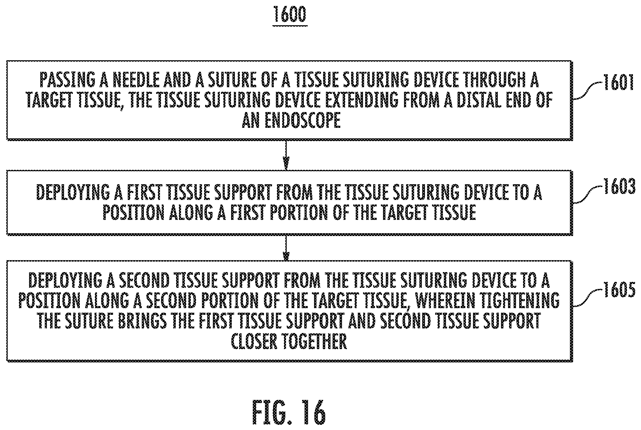

Turning now to **FIG. 16****,** a method 1600 according to embodiments of the present disclosure will be described in greater detail. At block 1601, the method may include passing a needle and a suture of a tissue suturing device through a target tissue, the tissue suturing device extending from a distal end of an endoscope. In some embodiments, the plurality of tissue supports are housed within a deployment device, wherein a first tissue support is exposed within a suture cavity. In some embodiments, the deployment device may be a cartridge or collar. In some embodiments, a cover may extend across the deployment device, the cover including an opening to permit access to the plurality of tissue supports by a needle. In some embodiments, the tissue supports may include one or more of the following: a flexible washer, an adhesive, a pledget, and an elastic or spring-loaded wire.

At block 1603, the method 1600 may include deploying a first tissue support from the tissue suturing device to a position along a first portion of the target tissue. In some embodiments, the first tissue support may be engaged within the suture cavity by the needle to remove the first tissue support from the deployment device. At block 1605, the method 1600 may include deploying a second tissue support from the tissue suturing device to a position along a second portion of the target tissue, wherein tightening the suture brings the first tissue support and the second tissue support closer together.

Although the illustrative method 1600 is described above as a series of acts or events, the present disclosure is not limited by the illustrated ordering of such acts or events unless specifically stated. For example, some acts may occur in different orders and/or concurrently with other acts or events apart from those illustrated and/or described herein, in accordance with the disclosure. In addition, not all illustrated acts or events may be required to implement a methodology in accordance with the present disclosure. Furthermore, the method 1600 may be implemented in association with the formation and/or processing of structures illustrated and described herein as well as in association with other structures not illustrated.

Some embodiments may be described using the expression "coupled" and "connected" along with their derivatives. These terms are not intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

As used herein, the singular forms "a," "an," and "the" are intended to include the plural forms as well, unless the context clearly indicates otherwise. It will be further understood that the terms, "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

Furthermore, the terms "substantial" or "substantially," as well as the terms "approximate" or "approximately," can be used interchangeably in some embodiments, and can be described using any relative measures acceptable by one of skill. For example, these terms can serve as a comparison to a reference parameter, to indicate a deviation that will still provide the intended function. Although non-limiting, the deviation from the reference parameter can be, for example, in an amount of less than 1%, less than 3%, less than 5%, less than 10%, less than 15%, less than 20%, and so on.

## Claims

1. A tissue suturing device, comprising:
a deployment device; and
a plurality of tissue supports deployable from the deployment device towards a target tissue, wherein the plurality of tissue supports includes a central opening for receiving a suture during deployment;
wherein the deployment device comprises:
a housing surrounding the plurality of tissue supports;
**characterized by**
a cover extending across the housing, the cover including an opening for access to the plurality of tissue supports by a needle attached to the suture.

2. The tissue suturing device of claim 1, further comprising:
a fixed end secured to a distal end of an endoscope; and
a free end opposite the fixed end, wherein the deployment device is coupled to one of: the fixed end, and the free end.

3. The tissue suturing device of any of claim 1 or 2, wherein the deployment device comprises a collar extending from the fixed end, wherein the collar defines a collar opening aligned with a working channel of the endoscope, and wherein each of the plurality of tissue supports extends around an exterior of the collar prior to deployment.

4. The tissue suturing device of any of claims 1-3, wherein the housing comprises an interior channel and a biasing device for biasing the plurality of tissue supports within the interior channel, wherein the plurality of tissue supports are arranged end-to-end within the interior channel.

5. The tissue suturing apparatus of any of claims 1-4, wherein the plurality of tissue supports is contained within the deployment device prior to deployment.

6. The tissue suturing apparatus of any of claims 1-5, wherein the tissue suturing device further comprises a needle, wherein the needle engages a tissue support of the plurality of tissue supports to deploy the tissue support.

7. The tissue suturing apparatus of claim 5 or 6, wherein the deployment device comprises a mandrel defining a central channel, wherein each of the plurality of tissue supports is disposed concentrically around the mandrel.

8. The tissue suturing apparatus of any of claims 1-7, the suture comprising:
a core; and
a sheath, wherein the sheath is operable to expand radially from the core in an area adjacent the target tissue.

9. The tissue suturing apparatus of claim 8, wherein the sheath comprises a plurality of flexible strands.

10. The tissue suturing apparatus of any of claims 1-9, wherein the plurality of tissue supports comprises a flexible washer and/or a pledget.

11. The tissue suturing apparatus of any of claims 1-10, wherein the cover includes one or more membranes extending across the housing.

12. The tissue suturing apparatus of any of claims 1-11, wherein the cover includes a plurality of slats extending across a frame of the housing to hold the plurality of tissue supports in place.

13. The tissue suturing apparatus of any of claims 10-12, wherein the plurality of tissue supports are cylindrical washers disposed concentrically around an exterior surface of a mandrel, wherein the plurality of tissue supports are elastically expand to fit the mandrel in the deployment device, and after deployment, the plurality of tissue supports may shrink or constrict to effectively close around a suture.

14. The tissue suturing apparatus of any of claims 10-12, wherein each of the plurality of tissue supports is a washer-shaped support disposed around an exterior of a collar, wherein the collar defines a collar opening aligned with a working channel of the endoscope and the needle may pass in and out of the collar opening during suturing, wherein, while situated on the collar, the plurality of tissue supports are stretched for deployment over the needle and the suture, and each of the plurality of tissue supports 540 may be deployed separately.

## Patentansprüche

1. Gewebenähgerät, aufweisend:
eine Ausbringvorrichtung; und
mehrere Gewebestützen, die von der Ausbringvorrichtung zu einem Zielgewebe hin ausbringbar sind, wobei die mehreren Gewebestützen eine zentrale Öffnung zur Aufnahme eines Nahtmaterials während des Ausbringens aufweisen;
wobei die Ausbringvorrichtung aufweist:
ein Gehäuse, das die mehreren Gewebestützen umgibt;
**gekennzeichnet durch**
eine Abdeckung, die sich über das Gehäuse erstreckt, wobei die Abdeckung eine Öffnung aufweist, um Zugang zu den mehreren Gewebestützen durch eine Nadel zu ermöglichen, die an dem Nahtmaterial angebracht ist.

2. Gewebenähgerät nach Anspruch 1, ferner aufweisend:
ein festes Ende, das an einem distalen Ende eines Endoskops befestigt ist; und
ein freies Ende, das dem festen Ende gegenüberliegt, wobei die Ausbringvorrichtung verbunden ist mit einem der folgenden:
dem festen Ende oder dem freien Ende.

3. Gewebenähgerät nach einem der Ansprüche 1 oder 2, wobei die Ausbringvorrichtung einen sich von dem festen Ende aus erstreckenden Kragen aufweist, wobei der Kragen eine Kragenöffnung definiert, die mit einem Arbeitskanal des Endoskops ausgerichtet ist, und wobei sich jede der mehreren Gewebestützen vor dem Ausbringen um eine Außenseite des Kragens erstreckt.

4. Gewebenähgerät nach einem der Ansprüche 1 - 3, wobei das Gehäuse einen inneren Kanal und eine Vorspannvorrichtung zum Vorspannen der mehreren Gewebestützen innerhalb des inneren Kanals aufweist, wobei die mehreren Gewebestützen Ende-zu-Ende innerhalb des inneren Kanals angeordnet sind.

5. Gewebenähgerät nach einem der Ansprüche 1 - 4, wobei die mehreren Gewebestützen vor dem Ausbringen in der Ausbringvorrichtung enthalten sind.

6. Gewebenähgerät nach einem der Ansprüche 1 - 5, wobei das Gewebenähgerät ferner eine Nadel aufweist, wobei die Nadel in eine Gewebestütze der mehreren Gewebestützen eingreift, um die Gewebestütze auszubringen.

7. Gewebenähgerät nach Anspruch 5 oder 6, wobei die Ausbringvorrichtung einen Dorn aufweist, der einen zentralen Kanal definiert, wobei jede der mehreren Gewebestützen konzentrisch um den Dorn angeordnet ist.

8. Gewebenähgerät nach einem der Ansprüche 1-7, wobei das Nahtmaterial aufweist:
einen Kern; und
eine Hülle, wobei die Hülle so betriebswirksam ist, dass sie sich radial von dem Kern in einem Bereich neben dem Zielgewebe ausdehnt.

9. Gewebenähgerät nach Anspruch 8, wobei die Hülle mehrere flexible Stränge aufweist.

10. Gewebenähgerät nach einem der Ansprüche 1 - 9, wobei die mehreren Gewebestützen jeweils eine flexible Unterlegscheibe und/oder ein Pledget aufweisen.

11. Gewebenähgerät nach einem der Ansprüche 1 - 10, wobei die Abdeckung eine oder mehrere Membranen aufweist, die sich über das Gehäuse erstrecken.

12. Gewebenähgerät nach einem der Ansprüche 1 - 11, wobei die Abdeckung mehrere Lamellen aufweist, die sich über einen Rahmen des Gehäuses erstrecken, um die mehreren Gewebestützen in Position zu halten.

13. Gewebenähgerät nach einem der Ansprüche 10 - 12, wobei die mehreren Gewebestützen zylindrische Unterlegscheiben sind, die konzentrisch um eine Außenfläche eines Dorns angeordnet sind, wobei die mehreren Gewebestützen elastisch ausdehnbar sind, um sich an den Dorn in der Ausbringvorrichtung anzupassen, und wobei sich die mehreren Gewebestützen nach dem Ausbringen zusammenziehen oder verengen können, um sich effektiv um ein Nahtmaterial zu schließen.

14. Gewebenähgerät nach einem der Ansprüche 10 - 12, wobei jede der mehreren Gewebestützen eine scheibenförmige Stütze ist, die um einen Außenbereich eines Kragens angeordnet ist, wobei der Kragen eine Kragenöffnung definiert, die mit einem Arbeitskanal des Endoskops ausgerichtet ist, und sich die Nadel während des Nähvorgangs in die Kragenöffnung hinein- und aus dieser herausbewegen kann, wobei die mehreren Gewebestützen, während sie sich auf dem Kragen befinden, zum Ausbringen über die Nadel und das Nahtmaterial gedehnt werden und jede der mehreren Gewebestützen 540 separat ausgebracht werden kann.

## Revendications

1. Dispositif de suture de tissu comprenant :
un dispositif de déploiement ; et
une pluralité de supports de tissu déployables du dispositif de déploiement vers un tissu cible, dans lequel la pluralité de supports de tissu comprennent une ouverture centrale pour recevoir une suture pendant le déploiement ;
dans lequel le dispositif de déploiement comprend :
un boîtier entourant la pluralité de supports de tissu ;
**caractérisé par** :
un couvercle s'étendant sur le boîtier, le couvercle comprenant une ouverture pour permettre l'accès à la pluralité de supports de tissu par une aiguille fixée à la suture.

2. Dispositif de suture de tissu selon la revendication 1, comprenant en outre :
une extrémité fixe fixée à une extrémité distale d'un endoscope ;
et
une extrémité libre opposée à l'extrémité fixe, dans lequel le dispositif de déploiement est couplé à l'une parmi : l'extrémité fixe et l'extrémité libre.

3. Dispositif de suture de tissu selon l'une quelconque des revendications 1 ou 2, dans lequel le dispositif de déploiement comprend un collier s'étendant à partir de l'extrémité fixe, dans lequel le collier définit une ouverture de collier alignée avec un canal de travail de l'endoscope, et dans lequel chacun de la pluralité de supports de tissu s'étend autour d'un extérieur du collier avant le déploiement.

4. Dispositif de suture de tissu selon l'une quelconque des revendications 1 à 3, dans lequel le boîtier comprend un canal intérieur et un dispositif de sollicitation pour solliciter la pluralité de supports de tissu à l'intérieur du canal intérieur, dans lequel la pluralité de supports de tissu sont agencés bout à bout à l'intérieur du canal intérieur.

5. Dispositif de suture de tissu selon l'une quelconque des revendications 1 à 4, dans lequel la pluralité de supports de tissu sont contenus à l'intérieur du dispositif de déploiement avant le déploiement.

6. Dispositif de suture de tissu selon l'une quelconque des revendications 1 à 5, dans lequel le dispositif de suture de tissu comprend en outre une aiguille, dans lequel l'aiguille met en prise un support de tissu de la pluralité de supports de tissu pour déployer le support de tissu.

7. Dispositif de suture de tissu selon la revendication 5 ou 6, dans lequel le dispositif de déploiement comprend un mandrin définissant un canal central, dans lequel chacun de la pluralité de supports de tissu est disposé de manière concentrique autour du mandrin.

8. Dispositif de suture de tissu selon l'une quelconque des revendications 1 à 7, la suture comprenant :
un noyau ; et
une gaine, dans lequel la gaine peut être actionnée pour subir une expansion radiale à partir du noyau dans une zone adjacente au tissu cible.

9. Dispositif de suture de tissu selon la revendication 8, dans lequel la gaine comprend une pluralité de brins flexibles.

10. Dispositif de suture de tissu selon l'une quelconque des revendications 1 à 9, dans lequel la pluralité de supports de tissu comprend une rondelle flexible et/ou une compresse.

11. Dispositif de suture de tissu selon l'une quelconque des revendications 1 à 10, dans lequel le couvercle comprend une ou plusieurs membranes s'étendant sur le boîtier.

12. Dispositif de suture de tissu selon l'une quelconque des revendications 1 à 11, dans lequel le couvercle comprend une pluralité de lamelles s'étendant sur un cadre du boîtier pour maintenir la pluralité de supports de tissu en place.

13. Dispositif de suture de tissu selon l'une quelconque des revendications 10 à 12, dans lequel la pluralité de supports de tissu sont des rondelles cylindriques disposées de manière concentrique autour d'une surface extérieure d'un mandrin, dans lequel la pluralité de supports de tissu subissent une expansion élastique pour s'adapter au mandrin dans le dispositif de déploiement, et après le déploiement, la pluralité de supports de tissu peuvent se rétracter ou se contracter pour se refermer efficacement autour d'une suture.

14. Dispositif de suture de tissu selon l'une quelconque des revendications 10 à 12, dans lequel chacun de la pluralité de supports de tissu est un support en forme de rondelle disposé autour d'un extérieur d'un collier, dans lequel le collier définit une ouverture de collier alignée avec un canal de travail de l'endoscope et l'aiguille peut passer à l'intérieur et à l'extérieur de l'ouverture de collier pendant la suture, dans lequel, tout en étant situés sur le collier, la pluralité de supports de tissu sont étirés pour le déploiement sur l'aiguille et la suture, et chacun de la pluralité de supports de tissu 540 peut être déployé séparément.
